# EUROPEAN PATENT SPECIFICATION

(11) **EP 1 731 180 B1**
(45) Date of publication and mention of the grant of the patent: **11.10.2017**
(21) Application number: 05727265.0
(22) Date of filing: 31.03.2005
(51) Int. Cl.: A61L 31/14

(54) **ANTIADHESIVE KIT, PROCESS FOR PRODUCING THE SAME AND METHOD OF ADHESION PREVENTION**
ANTIADHÄSIVES KIT, VERFAHREN ZU SEINER HERSTELLUNG UND VERFAHREN ZUR VERHINDERUNG VON ADHÄSION
KIT ANTIADHÉSIF, PROCÉDÉ POUR PRODUIRE CELUI-CI ET PROCÉDÉ CONSISTANT À EMPÊCHER L'ADHÉRENCE

(30) Priority: 31.03.2004 JP 2004102638; 13.09.2004 JP 2004264902
(43) Date of publication of application: 13.12.2006
(73) Proprietor: NIPRO CORPORATION, Osaka-fu, 531-8510 (JP)
(72) Inventor: MORINAGA, Yukihiro, c/o NIPRO CORPORATION, Osaka-shi, Osaka 5318510 (JP); KAMIMURA, Ryosuke, c/o NIPRO CORPORATION, Osaka-shi, Osaka 5318510 (JP); MATSUDA, Kazuhisa, c/o NIPRO CORPORATION, Osaka-shi, Osaka 5318510 (JP); SHIRASU, Akio, c/o NIPRO CORPORATION, Osaka-shi, Osaka 5318510 (JP)
(74) Representative: Arth, Hans-Lothar
(86) International application number: PCT/JP2005/006369
(87) International publication number: WO 2005/094915

(56) References cited:
- EP-A1- 1 022 031
- JP-A- 2003 235 955
- S. Isoda ET AL: ""Sandwich Technique" via Right Ventricle Incision to Repair Postinfarction Ventricular Septal Defect", Journal of Cardiac Surgery, vol. 19, no. 2, 1 March 2004 (2004-03-01), pages 149-150, XP055075133, ISSN: 0886-0440, DOI: 10.1111/j.0886-0440.2004.04028.x

## Description

### Technical Field

The present invention relates to an adhesion preventive kit for preventing adhesion in guided regeneration therapy for an injured tissue.

### Background Art

In various surgical operations, excision of affected parts and repair of injured sites are often performed. In particular, in the case where various organs, such as a lung, heart, liver, brain, digestive apparatus, and gallbladder, are targeted, a membrane-like material that covers the tissue of the organ must be filled or used for prosthesis on the cut surface, the deficient part, or the like. If the treatment is insufficiently done, the patient will die because of dysfunction of the organ in some cases. Also, even if the function of the organ itself is narrowly maintained, the body fluid, digestive juice, contents, etc. that exudes or leaks from the organ may cause infection, attacks or erosions to other organs, resulting in a crisis of life.

Furthermore, adhesion between an injured or deficient portion in the above-described organ and a tissue located in the surrounding part thereof (hereinafter, also referred to as surrounding tissue) occurs in high frequencies. As a result, dysfunction of the organ may be induced with a lapse of time. In particular, in the cardiac surgery field, the pericardium is excised to perform a cardiac surgical operation, but reoperation may be required depending on conditions after the operation. In this case, adhesion of the pericardium to the heart and the surrounding tissue is caused, so that an operation of peeling the adhesion is required at the beginning. The operation is performed with the greatest care so as to minimize blood loss of the patient, so that it often takes 3 to 5 hours to perform for this peeling operation.

For the purpose of solving such various problems, prosthetic membranes to cover organs or tissues of the organs have been developed by using various materials. However, in the case where the above-described prosthetic membranes are made of a synthetic fiber or the like, insuficiency of biocompatibility causes various drawbacks such as excessive calcification, foreign body reaction, and inflammatory reaction. Even in the case of using a bioabsorbable material, such material as the material itself mediates adhesion between the injured or deficient tissue and another tissue corresponding thereto cannot be used.

In addition, the above-described material must be a material having a function to prevent adhesion. Examples of a material that meets these requirements include hyaluronic acid and gelatin. Since those materials are mainly extracted from living bodies such as animals and purified, they have good biocompatibility and are already coming into practical use in various medical fields including medicines. Examples thereof include an adhesion preventive membrane using hyaluronic acid (Patent Documents 1 to 4). Furthermore, S. Isoda ET AL studied the repair of postinfarction ventricular septal defect by a "sandwich technique" involving two anti-adhesive patches and a gelatin-resorcin-formal glue as biodegradable layer (""Sandwich Technique" via Right Ventricle Incision to Repair Postinfarction Ventricular Septal Defect",Journal of Cardiac Surgery, vol. 19, no. 2, 1 March 2004 (2004-03-01), pages 149-150).

However, membranes made of the above-described biodegradable material lacked strength enough to resist sutures when used as prosthetic membranes. The inventors of the present invention have solved such problems and developed an adhesion preventive membrane having excellent suture strength in which all materials of a prosthetic membrane are composed of biodegradable and absorbable materials, and patent applications for the membrane have filed (Patent Documents 5 to 9).

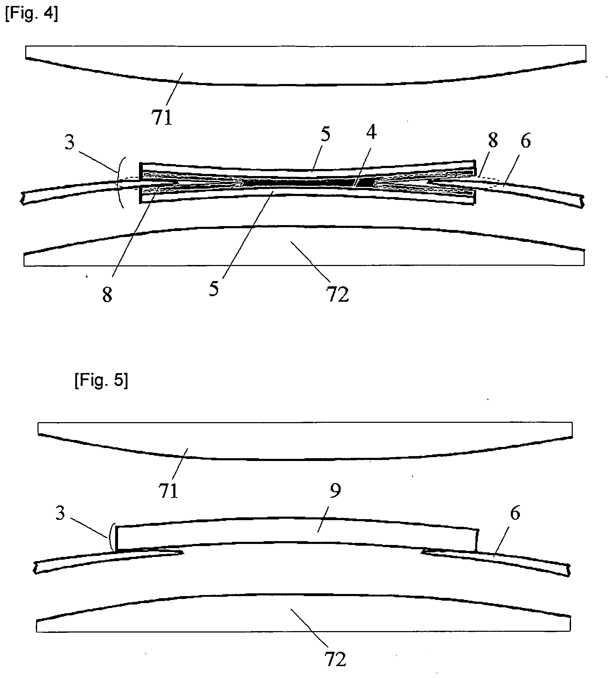

However, such adhesion preventive membranes were only used for prosthesis in the form shown in Fig. 5 for an injured or deficient portion. For example, in the case where injured or deficient portions of membrane-like, bag-like, and tube-like tissues are subjected to prosthesis, there is parts that directly come into contact with a surrounding tissue facing the opposite side of the surface subjected to prosthesis (A in Fig. 5), so that the edges of the injured tissue adhere to the surrounding tissue in some cases.

Patent Document 1: Japanese Laid-Open Patent Publication No. 61-234864
Patent Document 2: Japanese Laid-Open Patent Publication No. 06-073103
Patent Document 3: Japanese Laid-Open Patent Publication No. 08-157378
Patent Document 4: Japanese Laid-Open Patent Publication No. 08-333402
Patent Document 5: Japanese Laid-Open Patent Publication No. 2000-093497
Patent Document 6: Japanese Laid-Open Patent Publication No. 2000-210376
Patent Document 7: Japanese Laid-Open Patent Publication No. 2000-271207
Patent Document 8: Japanese Laid-Open Patent Publication No. 2003-235955
Patent Document 9: Japanese Laid-Open Patent Publication No. 2003-245351

### Disclosure

### Problems to be solved

Therefore, it is required to develop an adhesion preventive kit and a method of preventing adhesion, which enable prevention of adhesion even in a surrounding part of an edge of an injured or deficient tissue.

### Means for solving the Problems

The disclosure relates to the following:
(1) an adhesion preventive kit comprising the following (A) or (B):
   (A) a first membrane of at least two layers having a biodegradable base layer and an adhesion preventive layer provided respectively at outermost surfaces thereof and a second membrane of at least one layer having an adhesion preventive layer provided at an outermost surface thereof; or
   (B) an adhesion preventive membrane including a biodegradable base layer and an adhesion preventive layer, which membrane has an outermost surface constituted of the adhesion preventive layer and has a tissue sandwiching part,
(2) an adhesion preventive kit according to the item (1), wherein the biodegradable base layer contains collagen, polylactic acid, or polyglycolic acid,
(3) an adhesion preventive kit according to the item (1), wherein the biodegradable base layer is composed of woven cloth, a nonwoven fabric, a sheet, or a sponge,
(4) an adhesion preventive kit according to the item (1), wherein the biodegradable base layer is composed of a collagen nonwoven fabric,
(5) an adhesion preventive kit according to the item (1), wherein the adhesion preventive layer contains hyaluronic acid, collagen, or gelatin,
(6) an adhesion preventive kit according to the item (1), wherein the adhesion preventive layer is composed of a sheet or a sponge,
(7) an adhesion preventive kit according to the item (1), wherein the adhesion preventive layer is composed of a sponge of a mixture of collagen and hyaluronic acid,
(8) a method of producing an adhesion preventive kit which comprises (B) an adhesion preventive membrane including a biodegradable base layer and an adhesion preventive layer, which membrane has an outermost surface constituted of the adhesion preventive layer and has a tissue sandwiching part, wherein the method is characterized in that a surrounding part of the biodegradable base layer is branched in a normal line direction to a surface of the membrane,
(9) a method of producing an adhesion preventive kit according to the item (8), characterized by comprising: preparing a membrane of at least two layers having a biodegradable base layer and an adhesion preventive layer provided respectively at outermost surfaces thereof; layering the two membranes so that the biodegradable base layers face each other; and bonding or sewing only a central part thereof,
(10) a method of producing an adhesion preventive kit according to the item (8), characterized by comprising: layering two biodegradable base layers; bonding or sewing a central part thereof; and providing adhesion preventive layers at the outer surfaces of the biodegradable base layers,
(11) a method of preventing adhesion characterized by preventing adhesion between an injured or deficient tissue and a surrounding tissue located in a surrounding part of the injured or deficient tissue using an adhesion preventive kit comprising the following (A) or (B):
   (A) a first membrane of at least two layers having a biodegradable base layer and an adhesion preventive layer provided respectively at outermost surfaces thereof and a second membrane of at least one layer having an adhesion preventive layer provided at an outermost surface thereof; or
   (B) an adhesion preventive membrane including a biodegradable base layer and an adhesion preventive layer, which membrane has an outermost surface constituted of the adhesion preventive layer and has a tissue sandwiching part,
(12) a method of preventing adhesion according to the item (11), wherein the tissue is pericardium, pleura, diaphragm, cerebral dura mater, stomach, esophagus, or a digestive apparatus,
(13) a method of preventing adhesion according to the item (11), wherein the tissue is pericardium, and the surrounding tissue is heart,
(14) a method of preventing adhesion according to the item (11) characterized by separating the injured or deficient tissue from the surrounding tissue by at least the adhesion preventive layer, and
(15) a use of an adhesion preventive kit comprising the following (A) or (B):
   (A) a first membrane of at least two layers having a biodegradable base layer and an adhesion preventive layer provided respectively at outermost surfaces thereof and a second membrane of at least one layer having an adhesion preventive layer provided at an outermost surface thereof or
   (B) an adhesion preventive membrane including a biodegradable base layer and an adhesion preventive layer, which membrane has an outermost surface constituted of the adhesion preventive layer and has a tissue sandwiching part.

### Effect of the Invention

According to the present invention, the frequency and degree of adhesion are further reduced compared with a conventional adhesion preventive membrane, so that the time for an adhesion-peeling operation is reduced in reoperation. That is, the labor of an operator required in the surgery is reduced, and the blood loss of a patient is significantly suppressed during the surgery, thereby reducing patient burden.

### Brief Description of the Drawings

[Fig. 1] A conceptional illustration of a surgical operation in the case of using an adhesion preventive kit of a first embodiment of the present invention.
[Fig. 2] A conceptional illustration of a different surgical operation from that in Fig. 1 in the case of using an adhesion preventive kit of a first embodiment of the present invention.
[Fig. 3] A conceptional illustration of an adhesion preventive kit of a second embodiment of the present invention.
[Fig. 4] A conceptional illustration of a surgical operation mode in the case of using an adhesion preventive kit of a second embodiment of the present invention.
[Fig. 5] A conceptional illustration of a surgical operation in the case of using a conventional adhesion preventive membrane.
[Fig. 6] A photograph of the pericardium-defective site of the beagle in Example 2.
[Fig. 7] A photograph of the second membrane arranged on the surface of the heart of the beagle.
[Fig. 8] A photograph of the first membrane sutured to the pericardium of the beagle.
[Fig. 9] A photograph of the portion between the pericardium and the heart after three months from embedment of an adhesion preventive kit of the present invention in the pericardium-defective site of the beagle.
[Fig. 10] A photograph of the first membrane sutured to the pericardium of the male beagle in Comparative Example 1.
[Fig. 11] A photograph of the portion between the pericardium and heart after three months from embedment of the first membrane in the pericardium-defective site of the male beagle in Comparative Example 1 (adhesion ratio: 80%).
[Fig. 12] A photograph of the portion between the pericardium and the heart after three months from embedment of the first membrane in the pericardium-defective site of the male beagle in Comparative Example 1 (adhesion ratio: 50%).
[Fig. 13] A photograph of the commercially available ePTFE membrane sutured to the pericardium of the male beagle in Comparative Example 2.
[Fig. 14] A photograph of the portion between the pericardium and the heart after three months from application of the commercially available ePTFE membrane to the pericardium-defective site of the male beagle in Comparative Example 2.
[Fig. 15] A photograph of the adhesion preventive kit of the second embodiment of the present invention when viewed from an angle (in tissue sandwiching parts, the portions parallel to the Y-axis are bent to the Z-axis direction).
[Fig. 16] A photograph of an adhesion preventive kit of a second embodiment of the present invention in Fig. 15 when viewed from the XZ-plane.
[Fig. 17] A photograph of the adhesion preventive kit in Fig. 16, in which, in tissue sandwiching parts, the portions parallel to the X-axis are bent to the Z-axis direction.

### Description of Reference Numerals

1 First membrane of a first embodiment (A)
2 Second membrane of a first embodiment (A)
3 Membrane of a second embodiment (B)
4 Biodegradable base layer
5 Adhesion preventive layer
6 Injured or deficient tissue
7 Surrounding tissue
8 Tissue sandwiching part
9 Conventional adhesion preventive membrane

### Best Mode for carrying out the Invention

The term "adhesion preventive kit" of the present invention refers to one to be used for preventing adhesion between an injured or deficient tissue and a surrounding tissue located in a position physically accessible thereto when an incised or excised tissue is intended for prosthesis or regeneration in regenerative or surgical operations for a tissue injured by an accident or the like.

The term "tissue" as used in the present invention refers to an organ in a living body, a membrane tissue that cover the organ, and an apparatus. Examples of the organ include heart, liver, stomach, pancreas, gallbladder, and brain. Examples of the membrane tissue that covers the organ include pericardium, pleura, peritoneum, diaphragm, and cerebral dura mater. Examples of the apparatus include trachea, esophagus, and digestive apparatus.

The term "injury" as used in the present invention refers to a state where a tissue is damaged, and examples thereof include, but are not limited to, accidental injury caused by a foreign matter from outside of a body and surgical injury due to incision or the like in surgical operations. The term "deficiency" as used refers to a state where part of a tissue is lacking, and examples thereof include, but are not limited to, surgical deficiency caused by excision of an affected part in surgical operations.

The term "surrounding tissue" as used in the present invention refers to a tissue that is located in a surrounding part of the above-described injured or deficient tissue and is present at a position where adhesion may occur in regeneration of the above-described injured or deficient tissue. For example, in the case where the injured or deficient tissue is pericardium, the surrounding tissue thereof is heart, breastbone, and lung. In the case where the injured or deficient tissue is an apparatus such as trachea, esophagus, or digestive apparatus, the tissue also includes a wall surface facing an injured or deficient site in the apparatus.

The adhesion preventive kit of the present disclosure comprises the following two embodiments (A) and (B), and a person skilled in the art may select both embodiments.
(A) a first membrane of at least two layers having a biodegradable base layer and an adhesion preventive layer provided respectively at outermost surfaces thereof and a second membrane of at least one layer having an adhesion preventive layer provided at an outermost surface thereof; or
(B) an adhesion preventive membrane including a biodegradable base layer and an adhesion preventive layer, which membrane has an outermost surface constituted of the adhesion preventive layer and has a tissue sandwiching part.

The first embodiment (A) of an adhesion preventive kit of the present disclosure refers to a form that includes a first membrane of at least two layers having a biodegradable base layer and an adhesion preventive layer provided respectively at outermost surfaces thereof and a second membrane of at least one layer having an adhesion preventive layer provided at an outermost surface thereof. Depending on an injured or deficient tissue to be subjected to guided regeneration of the tissue, for example, in the case of guided regeneration of the pericardium excised in heart surgery operations, each of the above-described first and second membranes has an area of 1 to 200 cm², preferably 15 to 150 cm² and has a total thickness of 0.1 to 30 mm, preferably 0.5 to 8 mm, but the area and thickness are not limited thereto.

The above-described first membrane refers to a membrane that includes at least a biodegradable base layer and an adhesion preventive layer and is a membrane of at least two layers having the biodegradable base layer and adhesion preventive layer provided respectively at outermost surfaces thereof, which has effects on preventing adhesion between an injured or deficient tissue and a surrounding tissue and on promoting regeneration of the tissue in the regeneration of the injured or deficient tissue.

The above-described biodegradable base layer refers to a layer that has good biocompatibility when embedded in a living body, is made from a biodegradable and absorbable polymer to be degraded and absorbed after a certain period of time, and may enable guided regeneration of an injured or deficient tissue by using the biodegradable base layer as a scaffold. The term "biodegradable and absorbable polymer" refers to a material to be degraded and absorbed after a certain period of time from embedment in a living body. Examples thereof include collagen, polylactic acid, and polyglycolic acid. Collagen is preferable in view of excellent safety after embedded in a living body, adhesiveness to a cell, and a proliferative ability.

For the above-described collagen, collagen that has been subjected to a treatment so as to be able to dissolve in a solvent may be selected. Examples thereof include solubilized collagens such as an enzyme-solubilized collagen, an acid-solubilized collagen, an alkali-solubilized collagen, and a neutral-solubilized collagen. Among them, in view of ease of handling, an acid-solubilized collagen is preferable. Moreover, in view of safety when embedded in a living body, the collagen is preferably atelocollagen that has been subjected to a removal treatment for telopeptide, which is an antigenic determinant, but is not limited thereto. The collagen is derived from an extract from skin, tendon, bone, cartilage, organs, and the like of animal species such as oxen, pigs, bird, fish, rabbits, sheep, mouse, or humans. In view of availability, the collagen is preferably a collagen derived from pig skin, but is not limited thereto. Furthermore, examples of the type of collagen include types I, II, and III, and in view of ease of handling, the type is preferably types I and III, but is not limited thereto.

Examples of the form of a biodegradable base layer of the preset invention include a woven fabric, a nonwoven fabric, a sponge, and a sheet. Among them, in view of suture strength, a woven fabric and a nonwoven fabric are preferable. Moreover, in view of ease of production and cost, a nonwoven fabric is preferable, but is not limited thereto.

In the case of using the above-described woven or nonwoven fabric, a strand-like material to constitute such a form is produced. In view of improvement of suture strength, the outer diameter of the strand-like material is 0.01 to 1,000 µm, preferably 0.05 to 200 µm, more preferably 0.1 to 200 µm, but is not limited thereto.

The above-described strand-like material may be produced in accordance with a conventional method. For example, it may be produced by continuously spinning it out of a solution of the above-described biodegradable and absorbable polymer. In view of strand strength, the concentration of the above-described solution is 0.1 to 20 wt%, preferably 1 to 10 wt%, but is not limited thereto. Examples of a device to be used for discharging the solution include a gear pump, a dispenser, and various types of extruders. To perform a uniform spinning, the device is preferably a dispenser in view of stable discharge of a fixed amount of the solution with less pulsation, but is not limited thereto.

A solvent for a coagulation bath used in the wet spinning may be a solvent, suspension, emulsion, or solution that can coagulate the above-described biodegradable and absorbable polymer. In the case of using collagen as a raw material of a strand-like material, examples of the solvents include aqueous solutions of inorganic salts, inorganic salt-containing organic solvents, alcohols, and ketones. Examples of the aqueous solutions of inorganic salts include aqueous solutions of sodium sulfate, sodium chloride, ammonium sulfate, calcium chloride, and magnesium chloride. In particular, an aqueous solution of sodium chloride, sodium sulfate, or ammonium sulfate is preferable. A solvent obtained by dissolving or dispersing such inorganic salts in alcohols or acetones may also be used. Examples of the alcohols include methanol, ethanol, isopropanol, amyl alcohol, pentanol, hexanol, and ethylene glycol. Examples of the ketones include acetone and methyl ethyl ketone. Among them, in view of strength of a spun strand, ethanol, a solution of sodium chloride in ethanol, or a dispersion of sodium chloride in ethanol is preferably used, but the solvent is not limited thereto.

In the case where a woven fabric is selected as a biodegradable base layer, the distance between strand-like materials is 0.01 to 500 µm, preferably 0.1 to 200 µm, and each distance between the strand-like materials is preferably uniform but is not limited thereto.

The above-described woven fabric may be produced by a method using, for example, a weaving machine. Herein, in view of prevention of cutting in strand-like materials, the above-described strand-like materials are preferably modified into a twist yarn before use, but the form is not limited thereto.

In the case where a nonwoven fabric is selected as a biodegradable base layer, in view of suture strength, the distance between the strand-like materials is 0.01 to 500 µm, preferably 0.1 to 200 µm, but the materials may partially be in contact each other.

The above-described nonwoven fabric may have, for example, a form produced as a laminate that includes a first layer composed of a plurality of strand-like materials arranged substantially in parallel and a second layer composed of a plurality of strand-like materials arranged substantially in parallel such that the acute angle between the directions of arrangement of the strand-like materials is 70° to 90°. Furthermore, the nonwoven fabric may have a form that includes a third layer composed of a plurality of strand-like materials arranged substantially in parallel on the second layer such that the acute angle between the directions of the arrangement of strand-like materials in the second layer and those in the third layer is 70° to 90°. That is, in view of suture strength, the nonwoven fabric of the present invention preferably has a form that includes a layer composed of a plurality of strand-like materials arranged substantially in parallel (i.e., n layer; n is an integer of 2 or more) and upper and lower layers in contact with the n layer (i.e., n-1 layer or n+1 layer; n is an integer of 2 or more) such that the acute angle between the directions of the arranged strand-like materials in the n layer and those in the n-1 layer or n+1 layer is 70° to 90°, but the form is not limited thereto. In view of suture strength and the membrane weight, the total number of these layered articles is 2 to 20, preferably 4 to 16, but is not limited thereto. The nonwoven fabric having the above-described layered form is referred to as a layered nonwoven fabric in the present invention.

Moreover, the above-described sheet refers to a plane membrane having a surface that is nearly uniformly composed of the biodegradable and absorbable polymer. Examples of a method of producing them include extrusion molding, compression molding, and solvent casting methods, and in view of ease of production, the solvent casting method is preferable, but the method is not limited thereto.

Meanwhile, the above-described sponge refers to a substance in a porous form in which sections having many pores with uniform or nonuniform sizes are observed to be continuously or discontinuously dispersed in visual determination or under a microscope. An example of a method of forming thereof includes a method that involves casting a solution of a biodegradable polymer into a mold having an intended shape of a injured site in a tissue, followed by forming the sponge by means of a method such as air-drying, vacuum drying, freeze-thaw, vacuum freeze-drying. Among them, in view of uniform formation, the sponge is preferably formed by means of vacuum freeze-drying, but the method is not limited thereto. Examples of the above-described vacuum freeze-drying include, but are not limited to a method of drying a 0.05 to 30 wt% collagen solution at 10.67 Pa (0.08 Torr) or less in view of ease of production. After freeze-drying, the resultant is removed form the mold to give a sponge. Furthermore, in view of suture strength, the above-described sponge is preferably compressed by a press machine, but the process is not limited thereto.

In view of improvement of strength, the biodegradable base layer is preferably further immersed in a solution of a biodegradable and absorbable polymer and then air-dried, but the process is not limited thereto. Such a treatment is referred to as a binder treatment. In view of compatibility with the biodegradable base layer and strength, the biodegradable and absorbable polymer used in a binder treatment is preferably the same substance as that of the biodegradable base layer, but is not limited thereto. The binder treatment is performed by impregnating the above-described biodegradable base layer with a solution of a biodegradable and absorbable polymer or the like and drying it by an appropriate drying method such as air-drying, air-blast drying, drying under reduced pressure, drying at low temperature, or vacuum freeze-drying. A biodegradable base layer obtained by such binder treatment has drastically improved suture strength compared with an untreated biodegradable base layer. Meanwhile, in view of handling of the solution, the concentration of the biodegradable and absorbable polymer solution used in the binder treatment is 0.05 to 30 wt%, preferably 0.1 to 10 wt%, but is not limited thereto.

The biodegradable base layer of the present invention is most preferably a nonwoven fabric made of collagen strand in view of ease of production, suture strength, degradation resistance, and safety after embedded in a living body.

The term "adhesion preventive layer" as used in the present invention refers to a layer having a preventive effect on adhesion between an injured or deficient tissue and a surrounding tissue thereof in the regeneration of the tissue. An example of a raw material thereof includes a polymer having a preventive effect on adhesion. Examples thereof include collagen, gelatin, and hyaluronic acid, and the material is preferably hyaluronic acid having an excellent preventive effect on adhesion, and particularly preferably a mixture of collagen and hyaluronic acid that has improved degradation resistance.

The above-described hyaluronic acid may be derived from an animal or microorganism. Moreover, it may be used as a salt of an alkaline metal (e.g., sodium, potassium) or the like. Among them, from the viewpoint that the hyaluronic acid may be embedded in a living body, medical grade hyaluronic acid is preferable.

Meanwhile, in view of adhesion prevention and degradation resistance, a mixing ratio of collagen and hyaluronic acid is 3:7 to 7:3, preferably 5:5, but is not limited thereto.

Moreover, examples of the form of the above-described adhesion preventive layer include a sheet and a sponge. Among them, a sponge is preferable in view of ease in lamination on a biodegradable base layer. A method of producing a sheet or a sponge may be the same as the above-described method of producing a biodegradable base layer, but is not limited thereto.

The adhesion preventive layer of the present invention is most preferably in the form of sponge made of a mixture of collagen and hyaluronic acid in view of ease of production, adhesion preventive effect, degradation resistance, and safety after embedded in a living body.

Examples of the lamination method of a biodegradable base layer and an adhesion preventive layer in the above-described first membrane include a method of forming an adhesion preventive layer directly on a biodegradable base layer and a method of separately producing a biodegradable base layer and adhesion preventive layer and then laminating the layers. Among them, in view of ease of production, the method is preferably the method of separately producing a biodegradable base layer and adhesion preventive layer and then laminating the layers, but is not limited thereto.

An example of the above-described method of forming an adhesion preventive layer directly on a biodegradable base layer includes, but are not limited to, a method of forming an adhesion preventive layer by immersing a biodegradable base layer made of a nonwoven fabric in a solution of a biodegradable and absorbable polymer, followed by freeze-drying.

On the other hand, examples of the method of separately producing a biodegradable base layer and adhesion preventive layer and then laminating the layers include the above-described binder treatment and suture with thread. Among them, in view of ease of production, the method is preferably the above-described binder treatment using a biodegradable and absorbable polymer but is not limited thereto. The above-mentioned binder treatment may be performed in accordance with the above-described method, but is not limited thereto.

Examples of the above-described suture include a method using a commercially available surgical thread, the above-described strand-like material made from a biodegradable and absorbable polymer, and the like. Among them, in view of safety when embedded in a living body, it is preferable to sew with the above-described strand-like material made from a biodegradable and absorbable polymer, but is not limited thereto. In view of membrane strength, the sewing distance is 1 to 20 mm, preferably 2 to 10 mm, and the pitch distance of a sewing machine is 1 to 20 mm, preferably 2 to 10 mm, but the distances are not limited thereto.

The above-described biodegradable base layer and adhesion preventive layer are preferably further subjected to a crosslinking treatment, if necessary. The crosslinking treatment may enable timely control of the time to degrade the adhesion preventive layer in a living body. Examples of a crosslinking method include chemical crosslinking, gamma-irradiation, ultraviolet irradiation, electron beam irradiation, plasma irradiation, and crosslinking treatment by thermal dehydration. Among them, in the case where the above-described biodegradable base layer and adhesion preventive layer include collagen, in view of safety after embedded in a living body, the method is preferably the crosslinking treatment by thermal dehydration, but is not limited thereto. In the crosslinking treatment, degradability and absorbability in a living body can be controlled by crosslinking temperature and crosslinking time.

The above-described second membrane includes at least an adhesion preventive layer. The adhesion preventive layer may be a layer produced by the same method as that for the adhesion preventive layer in the above-described first membrane. Among them, in view of production cost, the form of the second membrane is preferably the same as that of the adhesion preventive layer in the above-described first membrane, but is not limited thereto.

In view of production cost, ease of production, membrane strength, safety when embedded in a living body, and the like, a preferable example of a first embodiment (A) of an adhesion preventive kit of the present invention is a form that includes a first membrane of at least two layers having a biodegradable base layer made of a collagen nonwoven fabric and an adhesion preventive layer made of sponge of a mixture of collagen and hyaluronic acid provided respectively at outermost surfaces thereof and a second membrane of at least one layer having an adhesion preventive layer made of sponge of a mixture of collagen and hyaluronic acid provided at an outermost surface thereof.

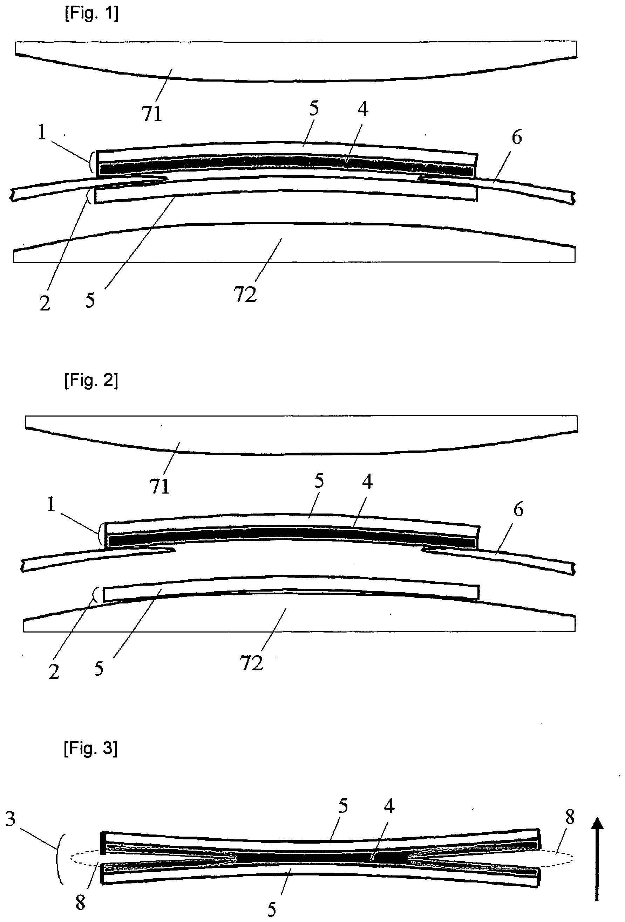

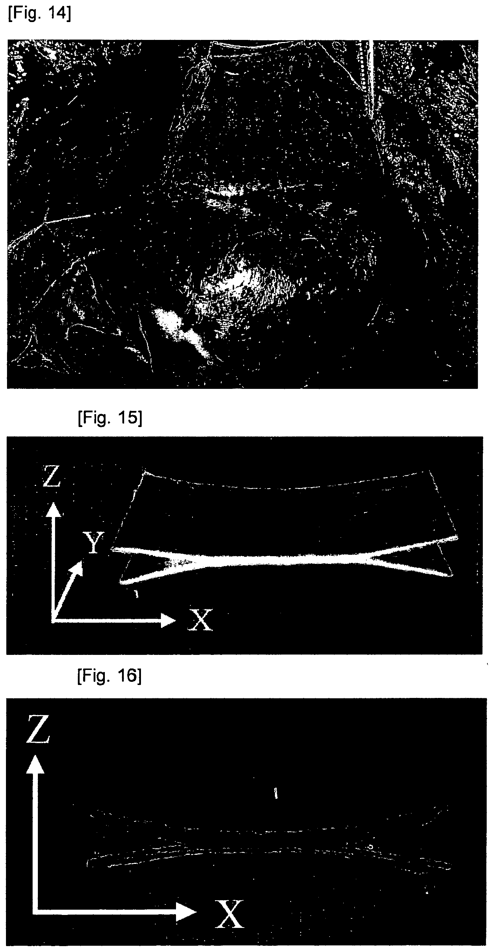

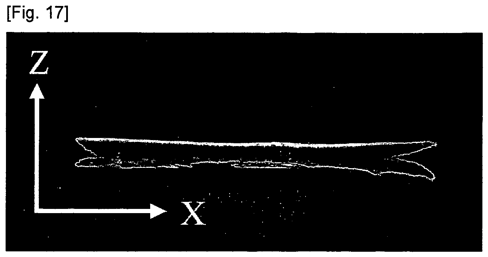

The second embodiment (B) of an adhesion preventive kit of the present invention is a form that includes an adhesion preventive membrane including a biodegradable base layer and an adhesion preventive layer, which membrane has an outermost surface constituted of the adhesion preventive layer and has a tissue sandwiching part. A conceptual illustration is shown in Fig. 3, and actual photographs are shown in Figs. 15 to 17. The area and thickness of the above-described membrane may be the same as those of the above-described first embodiment (A).

The biodegradable base layer and adhesion preventive layer in the second embodiment (B) may be layers produced by the same method as that for the biodegradable base layer and adhesion preventive layer in the above-described first embodiment (A), but are not limited thereto.

The term "tissue sandwiching part" as used in the present invention refers to one that sandwiches an edge of a tissue to protect the edge. The term "normal line direction to a surface of a membrane" refers to the arrow direction in Fig. 3 or the Z-axis directions in Figs. 15 to 17. With regard to the size of the sandwiching part, in view of strength of the membrane and ease in sandwiching a tissue, for example, in the case where the area of the surface of an adhesion preventive membrane is expressed as 100%, the area of a tissue sandwiching part is 0.5 to 90%, preferably 5 to 75%, more preferably 10 to 50% but is not limited thereto.

Examples of the method of producing the tissue sandwiching part of the present invention comprise:
(i) a method that includes the steps of laminating two membranes composed of two layers of a biodegradable base layer and an adhesion preventive layer so that the biodegradable base layers face each other; and bonding or sewing only the central part thereof,
(ii) a method that includes the steps of: laminating two membranes composed of a biodegradable base layer; bonding or sewing the central part thereof; and laminating an adhesion preventive layer on the outer surface of the biodegradable base layer,
(iii) a method that includes the steps of separately preparing a membrane-like material composed of a biodegradable base layer and an adhesion preventive layer and a membrane-like material composed of an adhesion preventive layer; and laminating these membranes and bonding or sewing the central part thereof,
(iv) a method that includes the steps of preparing a membrane having laminated adhesion preventive layers on the both surfaces of a biodegradable base layer; and making slits in an adhesion preventive layer, in a biodegradable base layer, or between an adhesion preventive layer and a biodegradable base layer in parallel with the surface of the membrane, and
(v) a method that includes the steps of making slits in a biodegradable base layer in parallel with the surface of the membrane; and laminating an adhesion preventive layer on the outer surface of the biodegradable base layer. Among them, in view of membrane strength and ease of production, the method is preferably (ii) a method that includes the steps of laminating two membranes composed of a biodegradable base layer; bonding or sewing the central part thereof and laminating an adhesion preventive layer on the outer surface of the biodegradable base layer, but is not limited thereto. The above-described membrane-like material composed of two layers of a biodegradable base layer and an adhesion preventive layer has the same structure as that of the first membrane in the first embodiment (A) of the present invention.

Each of the above-described lamination of a biodegradable base layer and an adhesion preventive layer, binder treatment, and suture may be performed in accordance with the above-described method described in the first embodiment (A), but is not limited thereto.

Moreover, the above-described slit may be accomplished by using a general-purpose instrument such as a microtome and knife.

In view of production cost, ease of production, membrane strength, safety when embedded in a living body, and the like, a particularly preferable example of the second embodiment (B) of an adhesion preventive kit of the present invention is a form that includes a membrane including a biodegradable base layer made of a collagen nonwoven fabric and an adhesion preventive layer made of a sponge of a mixture of collagen and hyaluronic acid, which membrane has an outermost surface constituted of the adhesion preventive layer and has a tissue sandwiching part at the surrounding part of the membrane.

The adhesion preventive kit of the present invention may provide a novel method of preventing adhesion between an injured or deficient tissue and a surrounding tissue thereof. Hereinafter, the method of preventing adhesion in the respective examples will be described with reference to drawings, but the present invention is not limited thereto.

Fig. 1 shows an example of the method of preventing adhesion using a first embodiment (A) of the adhesion preventive kit of the present invention. On one surface of an injured or deficient tissue 6, a first membrane 1 covers an injured or deficient portion so that a biodegradable base layer 4 in the first membrane is faced to the injured or deficient tissue 6 to make prosthesis for the part. Subsequently, a second membrane 2 is used for prosthesis for the other surface of the injured or deficient tissue 6. That is, the injured or deficient portion is sandwiched by the first membrane 1 and second membrane 2, which are arranged so that the injured or deficient portion is not exposed, so that the portion is not in physical contact with surrounding tissues 71 and 72, thereby providing a more efficient method of preventing adhesion than a conventional method. Herein, the positions of the first and second membranes may be reversed.

The example shown in Fig. 1 may be mainly applied to the case where a tissue such as pericardium, pleura, cerebral dura mater, diaphragm, stomach, esophagus, or digestive apparatus is injured or deficient, but is not limited thereto.

Fig. 2 shows another example of the method of preventing adhesion using the first embodiment (A) of the adhesion preventive kit of the present invention. In a surrounding tissue 71 of an injured or deficient tissue 6, a second membrane 2 is arranged at a position facing the injured or deficient portion. Subsequently, on the surface opposite to the surrounding tissue 71 of the injured or deficient tissue 6, a first membrane 1 covers the injured or deficient portion so that a biodegradable base layer 4 in the first membrane is faced to the injured or deficient tissue 6 to make prosthesis for the part. That is, the injured or deficient portion is not in physical contact with the surrounding tissues 71, 72, and the like, thereby providing a more efficient method of preventing adhesion than a conventional method.

The example shown in Fig. 2 may be mainly applied to the case where a tissue such as pericardium, pleura, diaphragm, or cerebral dura mater is injured or deficient, but is not limited thereto.

Fig. 4 shows an example of the method of preventing adhesion using the second embodiment (B) of the adhesion preventive kit of the present invention. An injured or deficient tissue 6 is held by a tissue sandwiching part 8. That is, the injured or deficient portion is not exposed, so that the portion is not in physical contact with surrounding tissues 71, 72, and the like, thereby providing a more efficient method of preventing adhesion than a conventional method.

The example shown in Fig. 4 may be mainly applied to the case where a tissue such as pericardium, pleura, cerebral dura mater, diaphragm, stomach, esophagus, or a digestive apparatus is injured or deficient, but is not limited thereto.

Therefore, the method of preventing adhesion using adhesion preventive kits of the first embodiment (A) and the second embodiment (B) of the present invention or the use thereof is characterized by separating an injured or deficient tissue from a surrounding tissue by the adhesion preventive layer.

### Examples

Hereinafter, the present invention will be described in detail by means of examples, but is not limited to these examples.

### Example 1: Preparation of adhesion preventive kit of first embodiment (A)

### (1) Preparation of adhesion preventive layer

Equal parts of an aqueous 1 wt% hydrofluoric acid solution and an aqueous 1 wt% collagen solution were mixed to give 250 mL of an equal part mixture of collagen and hydrofluoric acid. The mixture, which was in acidic state, was neutralized with an aqueous solution of 0.1 N sodium hydroxide, and then the resultant was loaded into a metallic container having a rectangular space (about 10 cm square), followed by freezed at -20°C for about 12 hours. Then, the frozen product was freeze-dried by using a lyophilizer (manufactured by EYELA Co.: FDU-830) under reduced pressure (13.32 Pa [0.1 Torr] or less) for about 24 hours and then compressed using a compressor (manufactured by Iuchi Seieido Co., Ltd.: 15 tons press machine) at a pressure of 100 kgf/cm². In this way, an adhesion preventive layer (about 10 cm square) made of sponge of a mixture of collagen and hyaluronic acid was obtained.

### (2) Preparation of biodegradable base layer

150 mL of an aqueous 7 wt% acid-solubilized collages solution was extruded into a coagulation bath containing 3 L of 99.5 vol% ethanol, dehydrated and coagulated, and then the resultant collagen strand was laminated in accordance with the method described in Japanese Laid-Open Patent Publication No. 2000-93497 to give a laminated collagen nonwoven fabric. Then, the resultant laminated collagen nonwoven fabric was air-dried in a clean bench, and subjected to a crosslinking reaction by thermal dehydration in a vacuum drying oven (manufactured by EYELA Co.: VOS-300VD) under reduced pressure (133.32.Pa [1 Torr] or less) at 120°C for 24 hours. After completion of the crosslinking reaction, an aqueous 1-wt% collagen solution was coated on the collagen nonwoven fabric as a binder treatment to fill the gaps between the filaments of the crosslinked laminated collagen nonwoven fabric, and then dried. After repeating the coating and drying operations three times, a crosslinking reaction using thermal dehydration is performed in a vacuum drying oven at 120°C for 12 hours. Subsequently, the resultant product was neutralized with an aqueous 7.5 wt% sodium bicarbonate solution, and air-dried in a clean bench to give a biodegradable base layer (about 10 cm square) made of a collagen nonwoven fabric of a prosthetic membrane for tissue regeneration.

### (3) Preparation of first membrane

By applying a neutralized solution containing a mixture of collagen and hyaluronic acid on the biodegradable base layer made of a collagen nonwoven fabric obtained in Example 1 (2), the adhesion preventive layer obtained in Example 1 (1) was bond to the biodegradable base layer. The resultant was air-dried in a clean bench and then performed a crosslinking reaction by thermal dehydration by heating it in a vacuum drying oven under reduced pressure (133.32 Pa [1 Torr] or less) at 110°C for 24 hours to give a first membrane.

### (4) Preparation of second membrane

The adhesion preventive layer prepared in Example 1 (1) was used as a second membrane without any modification.

### (5) Sterilization

The first membrane prepared in Example 1 (3) and the second membrane prepared in Example 1 (4) were subjected to gamma irradiation sterilization at an exposure dose of 25 kGy to give an adhesion preventive kit of the first embodiment of the present invention.

### Example 2: Adhesion prevention experiment using adhesion preventive kit of the present invention

### (1) Preparation of pericardium defect

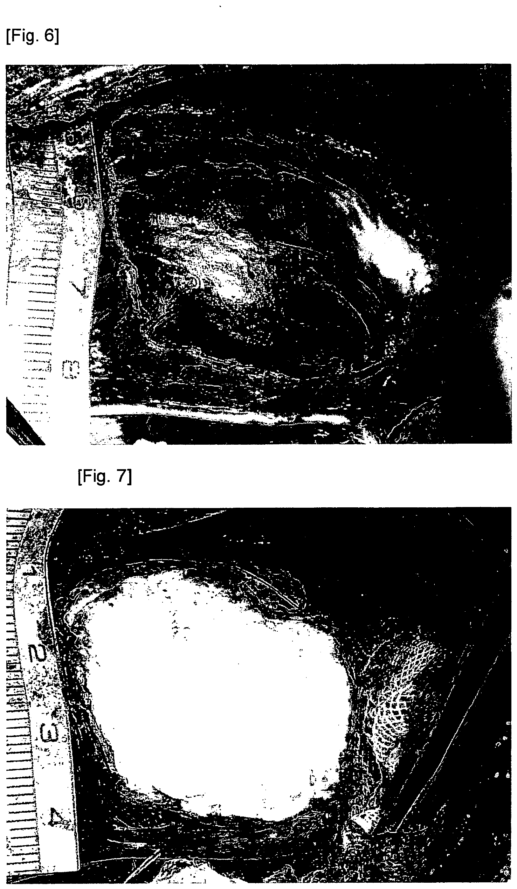

The thorax of each of six beagles (average body weight: about 10.5 kg) was opened between the left ribs under continuous anesthesia to expose the heart, and then about 4 cm square of the pericardium covering the surface of the heart was excised to prepare pericardium defect (Fig. 6).

### (2) Arrangement of second membrane

Next, the second membrane prepared in Example 1 (4) was trimmed to about 5 cm square and inserted through the pericardium-defective site, and the second membrane was arranged at a position facing the pericardium-defective site prepared in Example 2 (1) (Fig. 7).

### (3) Arrangement of first membrane

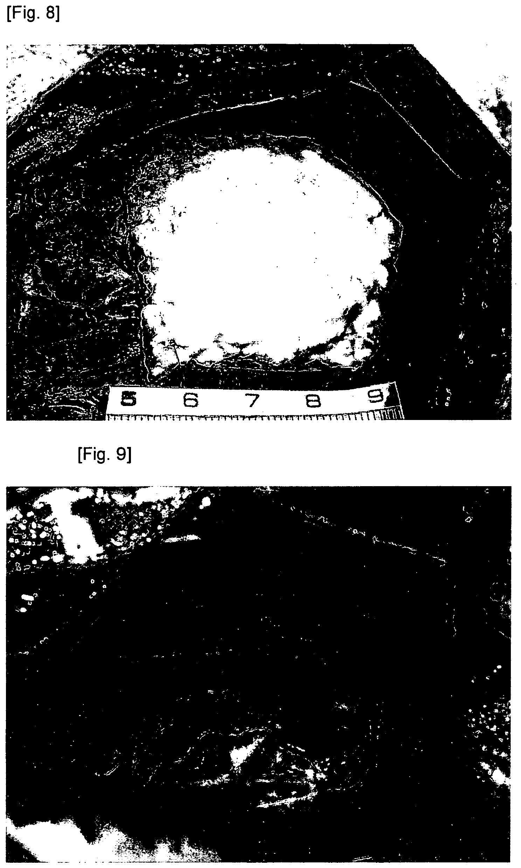

The first membrane prepared in Example 1 (3) was trimmed to about 5 cm square and immersed in sterilized distilled water for injection for 5 to 10 minutes to soften sufficiently. Then, the collagen nonwoven fabric in the first membrane was faced to the pericardium-defective side prepared in Example 2 (1), and the membrane was fixed by continuous suture (Fig. 8). Thereafter, the thorax was closed, and follow-up was performed for three months.

### Comparative Example 1: Adhesion prevention experiment using only first membrane of the present invention

### (1) Preparation of pericardium defect and arrangement of first membrane

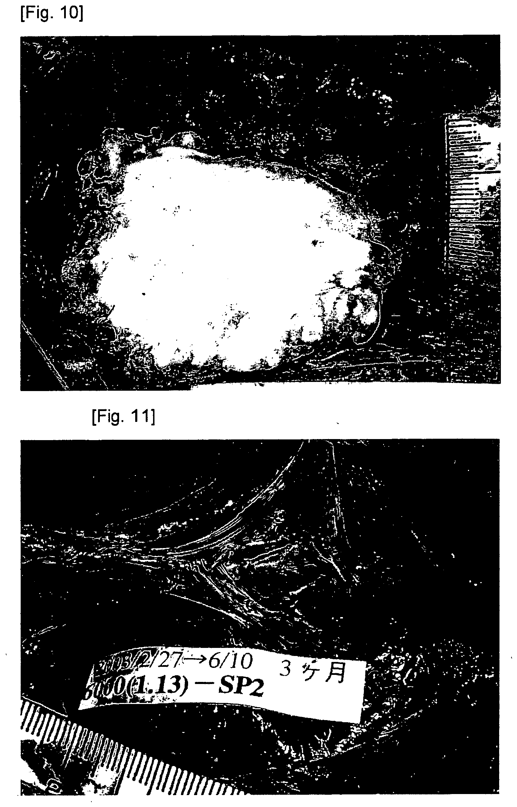

All the same procedures as in Example 2 were performed except that the step of Example 2 (2) was not performed. That is, only the procedures of Example 2 (1) and (3) were performed. Fig. 10 shows a photograph of the first membrane fixed on the pericardium-defective site by means of continuous suture in Comparative Example 1.

### Example 3: Statistical evaluation

During the follow-up for three months, all the six beagles of Example 2 and Comparative Example 1 had no particular abnormalities and were confirmed to maintain healthy conditions. After three months, the thoraxes were opened again by median incision, and the ratios of pericardium-heart adhesion area and the degree of adhesion were evaluated. Herein, the term "ratio of adhesion area" refers to a ratio of an area where adhesion occurred for the case that an area of a pericardium defect is expressed as 100%. Meanwhile, the degree of adhesion was judged by visual observation based on the determination criteria in Table 1.

**[Table 1]**

| Degree of adhesion | Grade |
|---|---|
| No adhesion | 0 |
| Manually peelable adhesion | 1 |
| Adhesion peelable by an instrument or the like | 2 |
| Adhesion that cannot be peeled without incision by an instrument or the like | 3 |

As a result, in Example 2, adhesion was observed in 2/6 cases. In both the two cases, the degrees of adhesion were grade 3, while the ratios of adhesion area were 10% in both the two cases. In other words, it may be said that an operator had little trouble in the adhesion peeling operation. On the other hand, in Comparative Example 1, adhesion was observed in 3 cases, and the degrees of adhesion were grade 3 in all the cases. However, the ratios of adhesion area were 50, 80, and 100%, respectively. From the above results, it is revealed that the adhesion preventive kit of the present invention has an excellent ability to prevent adhesion and has a function to regenerate pericardium in a pericardium defect. Evaluation results of adhesion-preventive effect are shown in Table 2.

**[Table 2]**

| Sample No. | Example 2 | | Comparative Example 1 | |
|---|---|---|---|---|
| | Ratio of adhesion area | Degree of adhesion | Ratio of adhesion area | Degree of adhesion |
| 1 | 0 | 0 | 80 | 3 |
| 2 | 0 | 0 | 0 | 0 |
| 3 | 10 | 3 | 100 | 3 |
| 4 | 10 | 3 | 50 | 3 |
| 5 | 0 | 0 | 0 | 0 |
| 6 | 0 | 0 | 0 | 0 |
| Average | 3.3 | 1 | 38.3 | 1.5 |

Moreover, a photograph of the adhesion state in Sample No. 1 of Example 2 is shown in Fig. 9. After the first and second membranes were degraded and absorbed, a pericardium-like membrane-like product was regenerated at the pericardium-defective site and there was no adhesion between the pericardium and heart.

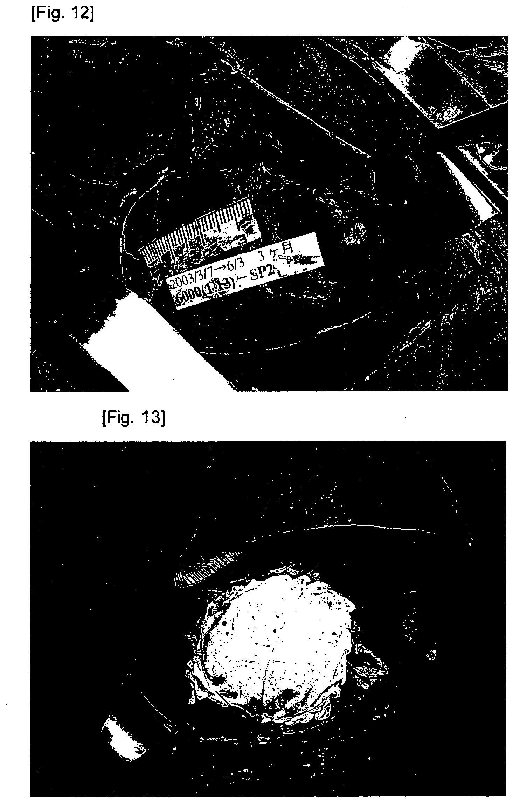

Photographs of adhesion states in Sample Nos. 1 and 4 of Comparative Example 1 are shown in Figs. 11 and 12, respectively. The first membrane was degraded and absorbed, and a pericardium-like membrane-like product was regenerated at the pericardium-defective site, but adhesions between the pericardium and heart were observed in some parts.

### Comparative Example 2: Adhesion prevention experiment using commercially available adhesion preventive membrane

### (1) Preparation of pericardium defect and arrangement of commercially available adhesion preventive membrane

The same procedures as in Comparative Example 1 were performed except that a commercially available adhesion preventive membrane (registered trademark: GORE-TEX), trimmed to about 5 cm square and made of expanded polytetrafluoroethylene (ePTFE), was used instead of the first membrane in Comparative Example 1. A photograph of the commercially available adhesion preventive membrane fixed on the pericardium-defective site by means of continuous suture in Comparative Example 2 is shown in Fig. 13.

### (2) Experimental results

During the follow-up for three months, the beagles had no particular abnormalities and were confirmed to maintain healthy conditions. After a lapse of three months, the thoraxes were opened again by median incision to perform evaluation, and GORE-TEX (registered trademark) was found to remain in each of the living bodies. Moreover, a severe degree of adhesion was formed between the pericardium and heart (Fig. 14).

### Example 3: Preparation of adhesion preventive kit of second embodiment (B)

In the same way as in Example 1 (2), two laminated collagen nonwoven fabric were prepared, each of which had been subjected to a binder treatment with an aqueous 1-wt% collagen solution. The aqueous collagen solution was separately coated on the 2 cm square of central parts of the binder-treated laminated nonwoven fabrics, and then the fabrics are bonded each other. Then, crosslinking by thermal dehydration was performed in a vacuum drying oven under high vacuum (133.32 Pa [1 Torr] or less) at 120°C for 12 hours to give a biodegradable base layer. Subsequently, the layer was immersed in an aqueous 7.5-wt% sodium bicarbonate solution for 30 minutes to perform a neutralization treatment, and washed with distilled water and air-dried in a clean bench to give a biodegradable base layer (10 cm square) having a tissue sandwiching part. A neutralized solution containing a mixture of collagen and hyaluronic acid was applied on the both surfaces of the biodegradable base layer, and the adhesion preventive layers obtained in Example 1 (1) were separately laminated on the both surfaces of membranes of the two biodegradable base layers. The product was air-dried in a clean bench and heated in a vacuum drying oven under high vacuum (133.32 Pa [1 Torr] or less) at 110°C for 24 hours to perform a crosslinking reaction by thermal dehydration to prepare an adhesion preventive kit of a second embodiment (B) of the present invention. Photographs of the kit are shown in Figs. 15 to 17.

### Industrial Applicability

The present invention is an innovative invention in surgery fields. Specifically, according to the present invention, the frequency and degree of adhesion are further reduced compared with a conventional adhesion preventive membrane, so that the time for an adhesion-peeling operation is drastically reduced in reoperation. That is, the labor of an operator required in the surgery is significantly reduced, and the blood loss of a patient is significantly suppressed in the surgery, thereby reducing patient burden.

## Claims

1. An adhesion preventive kit, comprising:
(A) a first membrane of at least two layers having a biodegradable base layer and an adhesion preventive layer provided respectively at outermost surfaces thereof and a second membrane of at least one layer having an adhesion preventive layer provided at an outermost surface thereof, wherein an injured or deficient portion is sandwiched by the first membrane and the second membrane; and the biodegradable base layer is faced to the injured or deficient tissue; or
(B) an adhesion preventive membrane including a biodegradable base layer and an adhesion preventive layer, which membrane has an outermost surface constituted of the adhesion preventive layer and has a tissue sandwiching part, wherein a surrounding part of the biodegradable base layer is branched in a normal line direction to a surface of the membrane,
wherein the biodegradable base layer contains collagen, polylactic acid, or polyglycolic acid.

2. The adhesion preventive kit according to Claim 1, wherein the biodegradable base layer is composed of woven cloth, a nonwoven fabric, a sheet, or a sponge.

3. The adhesion preventive kit according to Claim 1, wherein the biodegradable base layer is composed of a collagen nonwoven fabric.

4. The adhesion preventive kit according to Claim 1, wherein the adhesion preventive layer contains hyaluronic acid, collagen, or gelatin.

5. The adhesion preventive kit according to Claim 1, wherein the adhesion preventive layer is composed of a sheet or a sponge.

6. The adhesion preventive kit according to Claim 1, wherein the adhesion preventive layer is composed of a sponge of a mixture of collagen and hyaluronic acid.

7. A method of producing an adhesion preventive kit which comprises (B) an adhesion preventive membrane including a biodegradable base layer and an adhesion preventive layer, which membrane has an outermost surface constituted of the adhesion preventive layer and has a tissue sandwiching part, wherein the method is **characterized in that** a surrounding part of the biodegradable base layer is branched in a normal line direction to a surface of the membrane,
wherein the biodegradable base layer contains collagen, polylactic acid, or polyglycolic acid.

8. The method of producing an adhesion preventive kit according to Claim 7, **characterized by** comprising: preparing a membrane of at least two layers having a biodegradable base layer and an adhesion preventive layer provided respectively at outermost surfaces thereof; layering the two membranes so that the biodegradable base layers face each other; and bonding or sewing only a central part thereof.

9. The method of producing an adhesion preventive kit according to Claim 7, **characterized by** comprising: layering two biodegradable base layers; bonding or sewing a central part thereof; and providing adhesion preventive layers at the outer surfaces of the biodegradable base layers.

10. An adhesion preventive kit according to any one of claims 1 to 6 for use in a method of preventing adhesion, **characterized by** preventing adhesion between an injured or deficient tissue and a surrounding tissue located in a surrounding part of the injured or deficient tissue, comprising the following (A) or (B):
(A) a first membrane of at least two layers having a biodegradable base layer and an adhesion preventive layer provided respectively at outermost surfaces thereof and a second membrane of at least one layer having an adhesion preventive layer provided at an outermost surface thereof, wherein an injured or deficient portion is sandwiched by the first membrane and the second membrane; and the biodegradable base layer is faced to the injured or deficient tissue; or
(B) an adhesion preventive membrane including a biodegradable base layer and an adhesion preventive layer, which membrane has an outermost surface constituted of the adhesion preventive layer and has a tissue sandwiching part, wherein a surrounding part of the biodegradable base layer is branched in a normal line direction to a surface of the membrane,
wherein the biodegradable base layer contains collagen, polylactic acid, or polyglycolic acid.

11. The adhesion preventive kit according to Claim 10, wherein the tissue is pericardium, pleura, diaphragm, cerebral dura mater, stomach, esophagus, or a digestive apparatus.

12. The adhesion preventive kit according to Claim 10, wherein the tissue is pericardium, and the surrounding tissue is heart.

13. The adhesion preventive kit according to Claim 10, **characterized by** separating the injured or deficient tissue from the surrounding tissue by at least the adhesion preventive layer.

## Patentansprüche

1. Ein adhäsionsverilindemdes Kit umfassend:
(A) eine erste Membran aus mindestens zwei Schichten mit einer biologisch abbaubaren Basisschicht und einer an den jeweils äußersten Oberflächen vorgesehenen adhäsionsverhindernden Schicht und eine zweite Membran aus mindestens einer Schicht mit einer an einer äußersten Oberfläche vorgesehenen adhäsionsverhindernden Schicht, wobei ein verletzter oder mangelhafter Teil zwischen der ersten Membran und der zweiten Membran eingeschlossen ist; und die biologisch abbaubare Basisschicht dem verletzten oder mangelhaften Gewebe zugewandt ist; oder
(B) eine adhäsionsverhindernde Membran mit einer biologisch abbaubaren Basisschicht und einer adhäsionsverhindernden Schicht, die Membran eine äußere Oberfläche bestehend aus der adhäsionsverhindernden Schicht und einen Gewebe einbettenden Teil aufweist, wobei ein umgebender Teil der biologisch abbaubaren Basisschicht senkrecht zu einer Oberfläche der Membran verzweigt ist,
wobei die biologisch abbaubare Basisschicht Kollagen, Polymilchsäure oder Polyglykolsäure enthält,

2. Das adhäsionsverhindernde Kit gemäß Anspruch 1, wobei die biologisch abbaubare Basisschicht aus gewebtem Stoff, einem Vliesstoff, einer Folie oder einem Schwamm besteht.

3. Das adhäsionsverhindernde Kit gemäß Anspruch 1, wobei die biologisch abbaubare Basisschicht aus einem Kollagenvliesstoff besteht.

4. Das adhäsionsverhindernde Kit gemäß Anspruch 1, wobei die adhäsionsverhindernde Schicht Hyaluronsäure, Kollagen oder Gelatine enthält.

5. Das adhäsionsverhindernde Kit gemäß Anspruch 1, wobei die adhäsionsverhindernde Schicht aus einer Folie oder einem Schwamm besteht.

6. Das adhäsionsverhindernde Kit gemäß Anspruch 1, wobei die adhäsionsverhindernde Schicht aus einem Schwamm einer Mischung aus Kollagen und Hyaluronsäure besteht.

7. Ein Verfahren zur Herstellung eines adhäsionsverhindernden Kits, das (B) eine adhäsionsverhindernde Membran einschließlich einer biologisch abbaubaren Basisschicht und einer adhäsionsverhindernden Schicht umfasst, die Membran eine äußere Oberfläche bestehend aus der adhäsionsverhindernden Schicht und einen Gewebe einbettenden Teil aufweist, wobei das Verfahren **dadurch gekennzeichnet ist, dass** ein umgebender Teil der biologisch abbaubaren Basisschicht senkrecht zu einer Oberfläche der Membran verzweigt ist, wobei die biologisch abbaubare Basisschicht Kollagen, Polymilchsäure oder Polyglykolsäure enthält.

8. Das Verwahren zur Erstellung eines adhäsionsverhindernden Kits gemäß Anspruch 7, **dadurch gekennzeichnet, dass** es Herstellen einer Membran aus mindestens zwei Schichten mit einer biologisch abbaubaren Basisschicht und einer an den jeweils äußersten Oberfächen vargesehenen adhäsionsverhindernden Schicht; Schichten der zwei Membranen, so dass die biologisch abbaubaren Basisschichten einander zugewandt sind; und Kleben oder Nähren nur eines zentralen Teils davon, umfasst.

9. Das Verfahren zur Herstellung eines adhäsionsverhindernden Kits gemäß Anspruch 7, **dadurch gekennzeichnet, dass** es Schichten von zwei Membranen; Kleben oder Nähen eines zentralen Teils davon; und Bereitstellen adhäsionsverhindernder Schichten an den äußerten Oberflächen der biologisch abbaubaren Basisschichten, umfasst.

10. Ein adhäsionsverhinderndes Kit gemaß einem der Ansprüche 1 bis 6 zur Verwendung in einem Verfahren zur Verhinderung von Adhäsion, **gekennzeichnet durch** die Verhinderung der Adhäsion zwischen verletztem oder mangelhaftem Gewebe und umgebenden Gewebe, das sich in einem umgebenden Teil des verletzten oder mangelhaften Gewebes befindet, umfassend (A) oder (B):
(A) eine erste Membran aus mindestens zwei Schichten mit einer biologisch abbaubaren Basisschicht und einer an den jeweils äußersten Oberflächen vorgesehenen adhäsionsverhindernden Schicht und eine zweite Membran aus mindestens einer Schicht mit einer an einer äußersten Oberfläche vorgesehenen adhäsionsverhindernden Schicht, wobei ein verletzter oder mangelhafter Teil zwischen der ersten Membran und der zweiten Membran eingeschlossen ist; und die biologisch abbaubare Basisschicht dem verletzten oder mangelhaften Gewebe zugewandt ist; oder
(B) eine adhäsionsverhindernde Membran mit einer biologisch abbaubaren Basisschicht und einer adhäsionsverhindernden Schicht, die Membran eine äußere Oberfläche bestehend aus der adhäsionsverhindernden Schicht und einen Gewebe einbettenden Teil aufweist, wobei ein umgebender Teil der biologisch abbaubaren Basisschicht senkrecht zu einer Oberfläche der Membran verzweigt ist,
wobei die biologisch abbaubare Basisschicht Kollagen, Polymilchsäure oder Polyglykolsäure enthält.

11. Das adhäsionsverhindernde Kit gemäß Anspruch 10, wobei das Gewebe Perikard, Pleura, Zwerchfell, cerebrale Dura mater, Magen, Speiseröhre, oder ein Verdauungsapparat ist.

12. Das adhäsionsverhindernde Kit gemäß Anspruch 10, wobei das Gewebe Perikard und das umgebende Gewebe Herz ist.

13. Das adhäsionsverhindernde Kit gemäß Anspruch 10, **gekennzeichnet durch** Separierung des verletzten oder mangelhaften Gewebes von dem umgebenden Gewebe durch zumindest die adhäsionsverhindernde Schicht.

## Revendications

1. Un kit de prévention des adhérences, cumprenant :
(A) une première membrane d'au moins deux couches ayant une couche de base biodégradable et une couche de prévention des adhérences disposée sur les surfaces externes de celle-ci, et une seconde membrane d'au moins une couche ayant une couche de prévention des adhérences disposée sur la surface externe de celle-ci, dans lequel une partie lésée ou déficiente est prise en sandwich par la première et la deuxième membrane ; et la couche de base biodégradable est orientée vers le tissu lésé ou déficient ; ou
(B) une membrane de prévention des adhérences comprenant une couche de base biodégradable et une couche de prévention des adhérences, ladite membrane ayant une surface externe constituée par la couche de prévention des adhérences et ayant une partie prenant en sandwich le tissu, dans laquelle une partie entourant la couche de base biodégradable est ramifiée dans une direction de ligne normale à une surface de la membrane,
dans lequel la couche de base biodégradable contient du collagène, de l'acide polylactique ou de l'acide polyglycolique.

2. Le kit de prévention des adhérences selon la revendication 1, dans lequel la couche de base biodégradable est composée de tissu tissé, de tissu non tissé, d'une feuille ou d'une éponge.

3. Le kit de prévention des adhérences selon la revendication 1, dans lequel la couche de base biodégradable est composée d'un tissu non tissé de collagène.

4. Le kit de prévention des adhérences selon la revendication 1, dans lequel la couche de prévention des adhérences contient de l'acide hyaluronique, du collagène ou de la gélatine.

5. Le kit de prévention des adhérences selon la revendication 1, dans lequel la couche de prévention des adhérences est composée d'une feuille ou d'une éponge.

6. Le kit de prévention des adhérences selon la revendication 1, dans lequel la couche de prévention des adhérences est composée d'une éponge d'un mélange de collagène et d'acide hyaluronique.

7. Une méthode de fabrication d'un kit de prévention des adhérences comprenant (B) une membrane de prévention des adhérences comportant une couche de base biodégradable et une couche de prévention des adhérences, ladite membrane ayant une surface externe constituée par la couche de prévention des adhérences et ayant une partie prenant en sandwich le tissu, où la méthode est **caractérisée en ce qu'**une partie entourant la couche de base biodégradable est ramifiée dans une direction de ligne normale à une surface de la membrane, où la couche de base biodégradable contient du collagène, de l'acide polylactique ou de l'acide polyglycolique.

8. La méthode de fabrication d'un kit de prévention des adhérences selon la revendication 7, comprenant : préparer une membrane d'au moins deux couches ayant une couche de base biodégradable et une couche de prévention des adhérences disposée sur les surfaces externes de celle-ci ; superposer les deux membranes afin que les couches de base biodégradables soient disposées face à face ; et lier ou coudre uniquement une partie centrale de celles-ci.

9. La méthode de fabrication d'un kit de prévention des adhérences selon la revendication 7, comprenant : superposer deux couches de base biodégradables ; lier ou coudre une partie centrale de celles-ci; et disposer des couches de prévention des adhérences sur les surfaces externes des couches de base biodégradables.

10. Un kit de prévention des adhérences selon l'une quelconque des revendications 1 à 6 destiné à être utilisé dans une méthode de prévention des adhérences, **caractérisé en ce qu'**il empêche l'adhérence entre un tissu lésé ou déficient et un tissu environnant situé dans une partie environnante du tissu lésé ou déficient, comprenant les éléments suivants (A) ou (B) :
(A) une première membrane d'au moins deux couches ayant une couche de base biodégradable et une couche de prévention des adhérences disposée sur les surfaces externes de celle-ci, et une seconde membrane d'au moins une couche ayant une couche de prévention des adhérences disposée sur la surface externe de celle-ci, dans lequel une partie lésée ou déficiente est prise en sandwich par la première et la deuxième membrane ; et la couche de base biodégradable est orientée vers le tissu lésé ou déficient ; ou
(B) une membrane de prévention des adhérences comprenant une couche de base biodégradable et une couche de prévention des adhérences, ladite membrane ayant une surface externe constituée par la couche de prévention des adhérences et ayant une partie prenant en sandwich le tissu, dans laquelle une partie entourant la couche de base biodégradable est ramifiée dans une direction de ligne normale à une surface de la membrane,
dans lequel la couche de base biodégradable contient du collagène, de l'acide polylactique ou de l'acide polyglycolique.

11. Le kit de prévention des adhérences selon la revendication 10, dans lequel le tissu est le péricarde, la plèvre, le diaphragme, la dure-mère cérébrale, l'estomac, l'oesophage ou l'appareil digestif.

12. Le kit de prévention des adhérences selon la revendication 10, dans lequel le tissu est le péricarde et le tissu environnait est le cour.

13. Le kit de prévention des adhérences selon la revendication 10, **caractérisé en ce qu'**il sépare le tissu lésé ou déficient des tissus environnants au moins par la couche de prévention des adhérences.
